# EUROPEAN PATENT SPECIFICATION

(11) **EP 1 942 881 B1**
(45) Date of publication and mention of the grant of the patent: **08.08.2012**
(21) Application number: 06809750.0
(22) Date of filing: 05.10.2006
(51) Int. Cl.: A61K 31/131, A61K 31/135, A61P 13/12

(54) **COMPOSITIONS AND METHODS FOR TREATMENT OF RENAL FAILURE**
ZUSAMMENSETZUNGEN UND VERFAHREN ZUR BEHANDLUNG VON NIERENINSUFFIZIENZ
COMPOSITIONS ET METHODES DE TRAITEMENT DE L'INSUFFISANCE RENALE

(30) Priority: 06.10.2005 US 244150
(43) Date of publication of application: 16.07.2008
(73) Proprietor: Technion Research and Development of Foundation, Ltd., Technion City, 32000 Haifa (IL); RAPPAPORT FAMILY INSTITUTE FOR RESEARCH IN THE MEDICAL SCIENCES, Haifa 31096 (IL)
(72) Inventor: BINAH, Ofer, 36001 Nofit (IL); ABASSI, Zaid, A., 35156 Haifa (IL); YOUDIM, Moussa, B.H., P.O.B. 9649, 31096 Haifa (IL); BARAC, Yaron, 26364 Kiryat Motzkin (IL)
(74) Representative: Vossius & Partner
(86) International application number: PCT/IL2006/001170
(87) International publication number: WO 2007/039909

(56) References cited:
- WO-A-98/02152
- US-A- 5 486 541
- US-A- 5 786 390
- US-A1- 2004 176 469
- TORONYI, EVA ET AL: "Antiapoptotic effect of (-)-deprenyl in rat kidney after ischemia-reperfusion" MEDICAL SCIENCE MONITOR, vol. 8, no. 2, 2002, pages BR65-BR68, XP002429253
- ELIASH S ET AL: "Rasagiline and its (S) enantiomer increase survival and prevent stroke in salt-loaded stroke-prone spontaneously hypertensive rats." JOURNAL OF NEURAL TRANSMISSION (VIENNA, AUSTRIA : 1996) 2001, vol. 108, no. 8-9, 2001, pages 909-923, XP002429255 ISSN: 0300-9564
- FRIEDEWALD JOHN J ET AL: "Inflammatory cells in ischemic acute renal failure." KIDNEY INTERNATIONAL AUG 2004, vol. 66, no. 2, August 2004 (2004-08), pages 486-491, XP002429254 ISSN: 0085-2538
- FAURE C ET AL: "DIFFERENTIALY EXPRESSED GENES IN ISCHEMIC ACUTE RENAL FAILURE" MOLECULAR BIOLOGY OF THE CELL, BETHESDA, MD, US, vol. 9, 1998, page 473A, XP000953127 ISSN: 1059-1524
- ROBERT W. SCHRIER: "Atlas of Diseases of the Kidney" [Online] 1999, , XP002429257 Volume 1, Section II, Chapter 14: "Pathophysiology of Ischemic Acute Renal Failure" Retrieved from the Internet: URL:http://www.kidneyatlas.org/book/adk1_1 4.pdf and http://www.kidneyatlas.org/toc.htm> [retrieved on 2007-04-12] paragraph [0001] figure 14.1
- YOGEV-FALACH M ET AL: "The importance of propargylamine moiety in the anti-Parkinsonian drug resagiline and its derivatives in MAPK-dependent amyloid precursor protein processing" FASEB JOURNAL (FEDERATION OF AMERICAN SOCIETIES FOR EXPERIMENTAL BIOLOGY), BETHESDA, US, vol. 17, 2003, pages 2325-2327, XP002392213 ISSN: 0892-6638
- THADHANI R ET AL: "Acute renal failure", NEW ENGLAND JOURNAL OF MEDICINE, MASSACHUSETTS MEDICAL SOCIETY, BOSTON, MA, US, vol. 334, 30 March 1996 (1996-03-30), pages 1448-1460, XP003023605, ISSN: 1533-4406, DOI: DOI:10.1056/NEJM199605303342207

## Description

The present invention relates to compositions for the treatment of acute renal failure and, more particularly, to S-(-)-propargyl-1-aminoindan and pharmaceutically acceptable salts thereof for use in said compositions.

### Renal Failure

It is well established that the kidney plays a major role in the pathophysiology of congestive heart failure (CRF) (e.g., Dzau, 1987; Hillege *et al.,* 2000), and impaired renal function and reduced glomerular filtration rate (GFR) are considered a strong independent predictor of mortality in patients with CHF (Schrier *et al.,* 2001). Several, neurohumoral systems with renal vasoconstrictor properties such as sympathetic nervous system are thought to mediate the alterations in renal function in CHF (Dzau 1987; Hillege *et al.,* 2000). Sympathetic nerves, predominantly adrenergic, have been observed at all segments of the renal vasculature and tubule. Adrenergic nerve endings reach vascular smooth muscle and the mesangial cells of the juxtaglomerular apparatus, and all segments of the tubule: proximal, loop of Henle and distal. Consistent with these anatomical observations, stimulation of the renal nerve results in vasoconstriction of afferent and efferent arterioles, which subsequently results in reduction in renal blood flow and GFR.

. Pharmacologic evidence obtained in a variety of experimental animal models indicates that the renal vasoconstriction generated by the renal nerves is mediated by the activation of postjunctional α1-adrenoreceptors. Clinical and experimental investigations revealed that patients or animals with mild CHF have high plasma levels of norepinephrine. At the early stages, increased activity of sympathetic nervous system in CHF restores the hemodynamic abnormalities including hypoperfusion, diminished plasma volume, and impaired cardiac function by producing vasoconstriction and avid Na⁺ reabsorption. However, chronic excitation of this system induces several long-term adverse myocardial and renal effects (Abassi *et al.,* 2001). Bilateral renal denervation restored the natriuretic response to volume expansion, implicating increased renal sympathetic nerve activity in the Na⁺ avidity characteristic of CHF.

Activation of the sympathetic nervous system contributes to sodium retention, and to cardiac hypertrophy and fibrosis in clinical and experimental heart failure (Hostetter *et al.,* 1983; Abassi *et al.* 1990; Brodsky *et al*., 1998).

Acute renal failure (ARF) is a syndrome characterized by a sudden decrease in kidney function leading to a decrease or sudden loss of the ability of the kidneys to excrete wastes, concentrate urine, conserve electrolytes and maintain fluid balance. It is a frequent clinical problem, particularly in the intensive care unit, where it is associated with a mortality of between 50% and 80% (Schrier *et al,* 2004). ARF may occur following exposure to various therapeutic agents such as cyclosporine, aminoglycosides, nonsteroidal antiinflammatory drugs, cisplatin, amphotericin B, or procedures, e.g., radiocontrast media, or exposure to heavy metal, which inflict toxic and ischemic damage to the renal tissue (Green *et al,* 2000).

The mechanisms underlying ARF involve both vascular and tubular factors (Kribben *et al,* 1999). An ischemic insult to the kidney due to hypoperfusion will, in general, be the cause of the ARF. While a decrease in renal blood flow with diminished oxygen and substrate delivery to the tubule cells is an important ischemic factor, it must be remembered that a relative increase in oxygen demand by the tubule is also a factor in renal ischemia. Renal ischemia leads to a series of cellular events which might culminate in organ failure, depending on the cell type and the duration of ischemia. It was assumed that reperfusion, instituted before irreversible damage to the tissue occurred, would limit the insult to the organ. Contrary to such expectations, it was reported that reperfusion enhances renal damage (Canavese *et al.,* 1988). Several suggestions for the basis of this reperfusion injury' have been proposed. It has been linked to an attenuated restoration in renal blood flow which returns only to 60% of its preischemic value (Arendshorst *et al.,* 1975; Cristol *et al.,* 1993). Others have suggested that reactive oxygen species (ROS) generated during ischemia and reperfusion in the mitochondria, cause the damage (Canavese *et al.,* 1988; Greene *et al*., 1991).

Chronic renal failure (CRF) is the progressive loss of kidney function. The kidneys attempt to compensate for renal damage by hyperfiltration (excessive straining of the blood) within the remaining functional nephrons (filtering units that consist of a glomerulus and corresponding tubule). Over time, hyperfiltration causes further loss of function.

Chronic loss of function causes generalized wasting (shrinking in size) and progressive scarring within all parts of the kidneys. In time, overall scarring obscures the site of the initial damage. Yet, it is not until over 70% of the normal combine function of both kidneys is lost that most patients begin to experience symptoms of kidney failure.

### Propargylamine and propargylamine derivatives

Rasagiline, R-(+)-N-propargyl-1-aminoindan, a highly potent selective irreversible monoamine oxidase (MAO)-B inhibitor, has been shown to exhibit neuroprotective activity and antiapoptotic effects against a variety of insults in cell cultures and *in vivo*.

Rasagiline has been recently approved for treatment of Parkinson's disease in Europe, Israel, and in the U.S., under the name AZILECT® or AGILECT® (Teva Pharmaceutical Industries Ltd., Israel). The drug is effective with a dose as low as 1 mg/kg in monotherapy and as an adjunct to L-dopa, comparable in its effect to the anti-Parkinson catechol-O-methyltranferase (COMT) inhibitor, entacapone (Brooks and Sagar, 2003).

Rasagiline exhibits neuroprotective activities both *in vitro* and *in vivo* (for review see Mandel *et al*., 2003; Youdim *et al.,* 2003) which may contribute to its possible disease modifying activity. It is metabolized to its major metabolite aminoindan (TVP-136) (Youdim *et al.,* 2001), which also have neuroprotective activity against serum deprivation and 1-methamphetamine-induced neurotoxicity in partially differentiated PC-12 cells (Am *et al.,* 2004).

By contrast, selegiline (1-depranyl), a selective MAO-B inhibitor which is a useful anti-Parkinson drug both in monotherapy and as an adjunct to L-DOPA therapy, and has L-DOPA sparing action (Parkinson Study Group, 1989), is a propargyl derivative of 1-methamphetamine. Thus, the major metabolite of selegiline, 1-methamphetamine (Kraemer and Maurer, 2002; Shin, 1997), is neurotoxic. In contrast to aminoindan, 1-methamphetamine prevents the neuroprotective activities of rasagiline and selegiline in partially differentiated cultured PC-12 cells (Am *et al*., 2004).

Selegiline and methamphetamine, unlike rasagiline and aminoindan, have sympathomimetic activity (Simpson, 1978) that increases heart rate and blood pressure (Finberg *et al.,* 1990; Finberg *et al*., 1999). Recent studies (Glezer and Finberg, 2003) have indicated that the sympathomimetic action of selegiline can be attributed to its 1-methamphetamine and amphetamine metabolites. These properties are absent in rasagiline and in its metabolite aminoindan. Parkinsonian patients receiving combined treatments with selegiline plus levodopa have been reported to have a higher mortality rate than those treated with levodopa alone (Lees, 1995). This is not related to its MAO-B inhibitory activity, but rather attributed to its sympathomimetic action and methamphetamine metabolites (Lavian *et al.,* 1993).

Several propargylamine derivatives have been shown to selectively inhibit MAO-B and/or MAO-A activity and, thus to be suitable for treatment of neurodegenerative diseases such as Parkinson's and Alzheimer's disease. In addition, these compounds have been further shown to protect against neurodegeneration by preventing apoptosis.

R(+)-N propargyl-1-aminoindan and pharmaceutically acceptable salts thereof were first disclosed in US Patents US 5,387,612, US 5,453,446, US 5,457,133, US 5,576,353, US 5,668,181, US 5,786,390, US 5,891,923, and US 6,630,514 as useful for the treatment of Parkinson's disease, memory disorders, dementia of the Alzheimer type, depression, and the hyperactive syndrome. The 4-fluoro-, 5-fluoro- and 6-fluoro-N-propargyl-1-aminoindan derivatives were disclosed in US 5,486,541 for the same purposes.

US 5,519,061, US 5,532,415, US 5,599,991, US 5,744,500, US 6,277,886, US 6,316,504, US 5,576,353, US 5,668,181, US 5,786,390, US 5,891,923, and US 6,630,514 disclose R(+)-N-propargyl-1-aminoindan and pharmaceutically acceptable salts thereof as useful for treatment of additional indications, namely, an, affective illness, a neurological hypoxia or anoxia, neurodegenerative diseases, a neurotoxic injury, stroke, brain ischemia, a head trauma injury, a spinal trauma injury, schizophrenia, an attention deficit disorder, multiple sclerosis, and withdrawal symptoms.

US 6,251,938 describes N-propargyl-phenylethylamine compounds, and US 6,303,650, US 6,462,222 and US 6,538,025 describe N-propargyl-1-aminoindan and N-propargyl-1-aminotetralin compounds, said to be useful for treatment of depression, attention deficit disorder, attention deficit and hyperactivity disorder, Tourette's syndrome, Alzheimer's disease and other dementia such as senile dementia, dementia of the Parkinson's type, vascular dementia and Lewy body dementia.

The first compound found to selectively inhibit MAO-B was R-(-)-N-methyl-N-(prop-2-ynyl)-2-aminophenylpropane, also known as L-(-)-deprenyl, R-(-)-deprenyl, or selegiline. In addition to Parkinson's disease, other diseases and conditions for which selegiline is disclosed as being useful include: drug withdrawal (WO 92/21333, including withdrawal from psychostimulants, opiates, narcotics, and barbiturates); depression (US 4,861,800); Alzheimer's disease and Parkinson's disease, particularly through the use of transdermal dosage forms, including ointments, creams and patches; macular degeneration (US 5,242,950); age-dependent degeneracies, including renal function and cognitive function as evidenced by spatial learning ability (US 5,151,449); pituitary-dependent Crushing's disease in humans and nonhumans (US 5,192,808); immune system dysfunction in both humans (US 5,387,615) and animals (US 5,276,057); age-dependent weight loss in mammals (US 5,225,446); schizophrenia (US 5,151,419); and various neoplastic conditions including cancers, such as mammary and pituitary cancers. WO 92/17169 discloses the use of selegiline in the treatment of neuromuscular and neurodegenerative disease and in the treatment of CNS injury due to hypoxia, hypoglycemia, ischemic stroke or trauma. In addition, the biochemical effects of selegiline on neuronal cells have been extensively studied (e.g., see Tatton, *et al.,* 1991 and 1993). US 6,562,365 discloses the use of desmethylselegiline for selegiline-responsive diseases and conditions.

US 5,169,868, US 5,840,979 and US 6,251,950 disclose aliphatic propargylamines as selective MAO-B inhibitors, neuroprotective and cellular rescue agents. The lead compound, (R)-N-(2-heptyl)methyl-propargylamine (R-2HMP), has been shown to be a potent MAO-B inhibitor and anti-apoptotic agent (Durden *et al.,* 2000).

Propargylamine was reported many years ago to be a mechanism-based inhibitor of the copper-containing bovine plasma amine oxidase (BPAO), though the potency was modest. US 6,395,780 discloses propargylamine as a weak glycinecleavage system inhibitor. Copending US Patent Application No. 10/952,379 discloses that propargylamine exhibits neuroprotective and anti-apoptotic activities and can, therefore, be used for all known uses of rasagiline and similar drugs containing the propargylamine moiety.

Copending US Patent Application No. 10/952,367 of the present applicants discloses and claims a method for treatment of a cardiovascular disorder or disease which comprises administering to the subject an amount of an active agent selected from the group consisting of propargylamine, a propargylamine derivative, and a pharmaceutically acceptable salt thereof.

US 2004/0176469 A1 discloses a method of preventing, reducing .and reversing the toxic effects of anti-inflammatory drugs and enhance their beneficial effects, comprising administering to a subject an effective amount of deprenyl or propargylamine compounds (monoamine oxidase [MAO] inhibitors). The anti-inflammatory drug and MAO inhibitor can be chemically linked, physically mixed or administered separately.

In Eliash et al. (2001), salt-loaded stroke-prone spontaneously hypertensive rats (SHRSP) were used to determine whether chronic treatment with the selective MAO-inhibitor R-(+)-N-propargyl-1-aminoindan (rasagiline) or with its S-enantiomer can prevent or delay stroke or improve its outcome in these animals.

US 5,786,390 A discloses a pharmaceutical composition which consists essentially of a therapeutically effective amount of R-(+)-N-propargyl-1-aminoindan or a pharmaceutically acceptable salt thereof and a pharmaceutically acceptable carrier.

WO 98/02152 describes pharmaceutical compositions for the treatment of a neurological disorder or neurotrauma or for improving memory in a patient, comprising a therapeutically effective amount of S-(-)-N-propargyl-1-aminoindan or a pharmaceutically acceptable salt thereof as active ingredient, and a pharmaceutically active carrier.

US 5,486,541 A discloses N-propargyl-1-aminoindan compounds which are monofluorinated in the phenyl ring and their use as selective inhibitors of monoamine oxidase (MAO). There are provided several processes for the preparation of these novel compounds. There are also provided as novel compounds 1-aminoindans monofluorinated in the phenyl ring, which serve as intermediates in the preparation of the corresponding novel N-propargyl derivatives.

To the best of our knowledge, the renal effects of rasagiline and its metabolites were not disclosed nor examined.

We examined the acute and chronic effects of rasagiline and its metabolites on: (i) renal clearance and excretory function in rats with renal ischemia, and (ii) on renal functions in rats with experimental heart failure. We have then found that rasagiline, and to a greater extent its S-enantiomer S(-)-N-propargyl-1-aminoindan, produced significant diuretic and natriuretic responses in association with glomerular filtration rate (GFR) in rats with congestive heart failure, and induced diuretic and natriuretic responses accompanied by improved GFR in the ischemic kidney.

Thus, the present invention relates to:
1. Use of an active agent for the preparation of a pharmaceutical composition for treatment or prevention of acute renal failure (ARF), wherein said active agent is selected from S-(-)-N-propargyl-1-aminoindan, or a pharmaceutically acceptable salt thereof.
2. The use of above 1, wherein said active agent is S-(-)-N-propargyl-1-aminoindan.
3. The use of above 1, wherein said active ingredient is a pharmaceutically acceptable salt of S-(-)-N-propargyl-1-aminoindan.
4. The use of above 3, wherein said pharmaceutically acceptable salt is selected from the mesylate salt; the esylate salt; the sulfate salt; the maleate salt; the fumarate salt; the tartrate salt; the hydrobromide salt; the p-toluenesulfonate salt; the benzoate salt; the acetate salt; the phosphate salt; and the hydrochloride salt of S-(-)-N-propargyl-1-aminoindan.
5. The use of above 1, wherein said ARF is caused by congestive heart failure.
6. A pharmaceutically active agent selected from S-(-)-N-propargyl-1-aminoindan, or a pharmaceutically acceptable salt thereof, for use in treating or preventing acute renal failure (ARF).
7. The pharmaceutically active agent as defined in above 6 for the use as defined in above 6, wherein said ARF is caused by congestive heart failure.

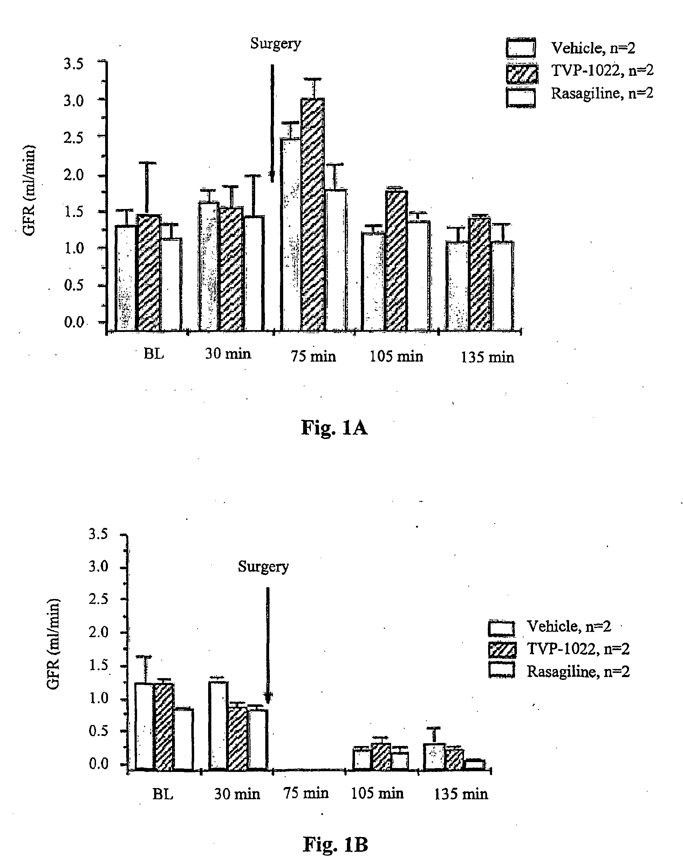

Figs. 1A-1B show the effects of pretreatment with rasagiline or S-(-)-N-propargyl-1-aminoindan (TVP-1022) (1 mg/kg), 30 minutes prior to the induction of renal ischemia in rats, and a sustained infusion of the drug at a dose of 2 mg/kg/h, on the glomerular filtration rate (GFR) of the normal (1A) and ischemic (1B) kidney. For induction of renal ischemia, the renal artery was clamped for forty-five minutes of ischemia followed by 60 min of reperfusion. Aninals treated with vehicle (saline) served as control (BL = basal level).

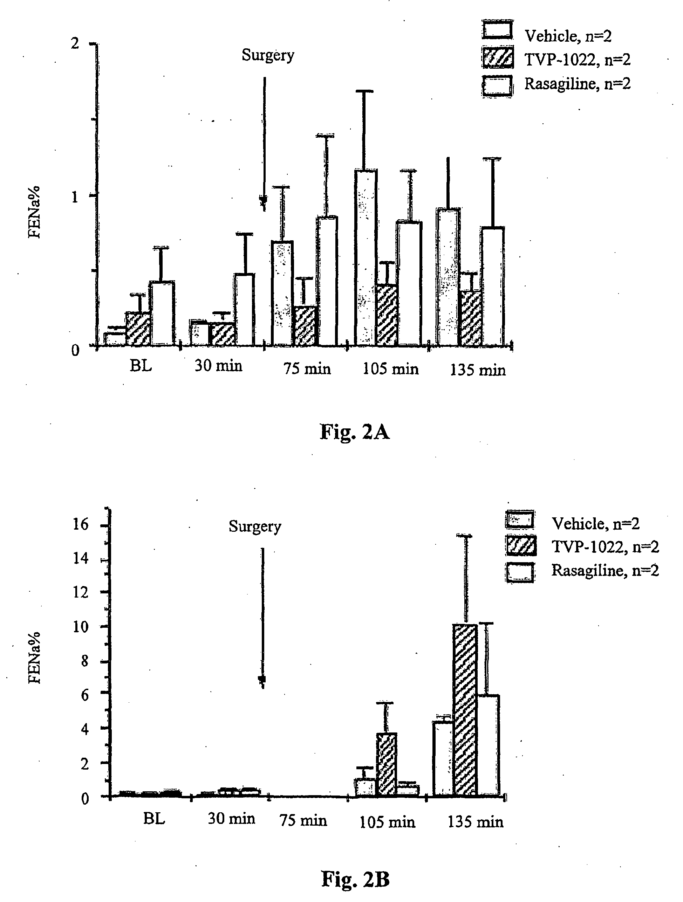

Figs. 2A-2B show the effects of pretreatment with rasagiline or S-(-)-N-propargyl-1-aminoindan (TVP-1022) (1 mg/kg), 30 minutes prior to the induction of renal ischemia in rats, and a sustained infusion of the drug at a dose of 2 mg/kg/h, on the fractional excretion of sodium (FENa) of the normal (2A) and ischemic (2B) kidney. For induction of renal ischemia, the renal artery was clamped for forty-five minutes of ischemia followed by 60 min of reperfusion. Aninals treated with vehicle (saline) served as control (BL = basal level).

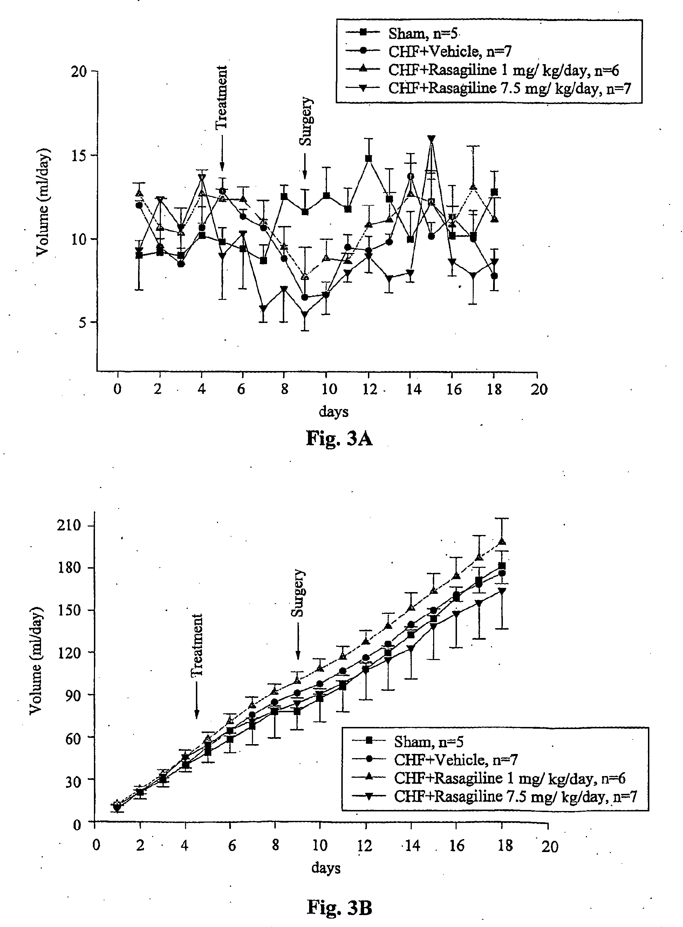

**Figs. 3A-3D** show the effects of chronic treatment with 1 or 7.5 mg/kg/day of either rasagiline or S-(-)-N-propargyl-1-aminoindan (TVP-1022) on daily (**3A, 3C**) and cumulative (**3B, 3D**) urine volume in rats with CHF vs. sham operated animals. Rasagiline or S-(-)-N-propargyl-1-aminoindan were given orally for 5 days to normal rats which were then subjected to either sham operation or creation of ACF as described in Example 2 hereinafter. Oral treatment continued for additional 14 days. Rats treated with vehicle (water) served as controls (P<0.05).

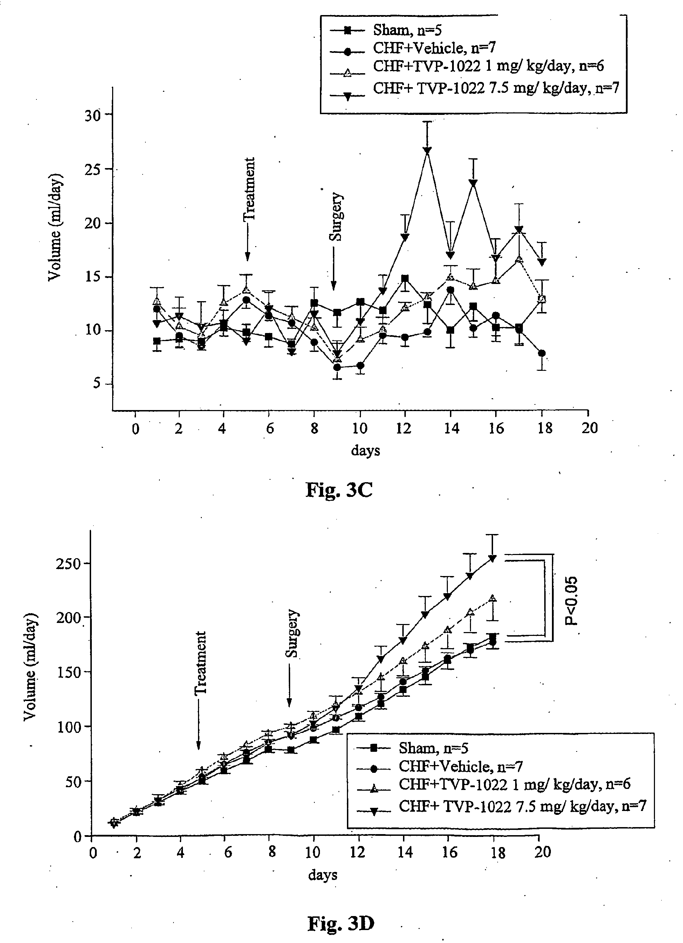

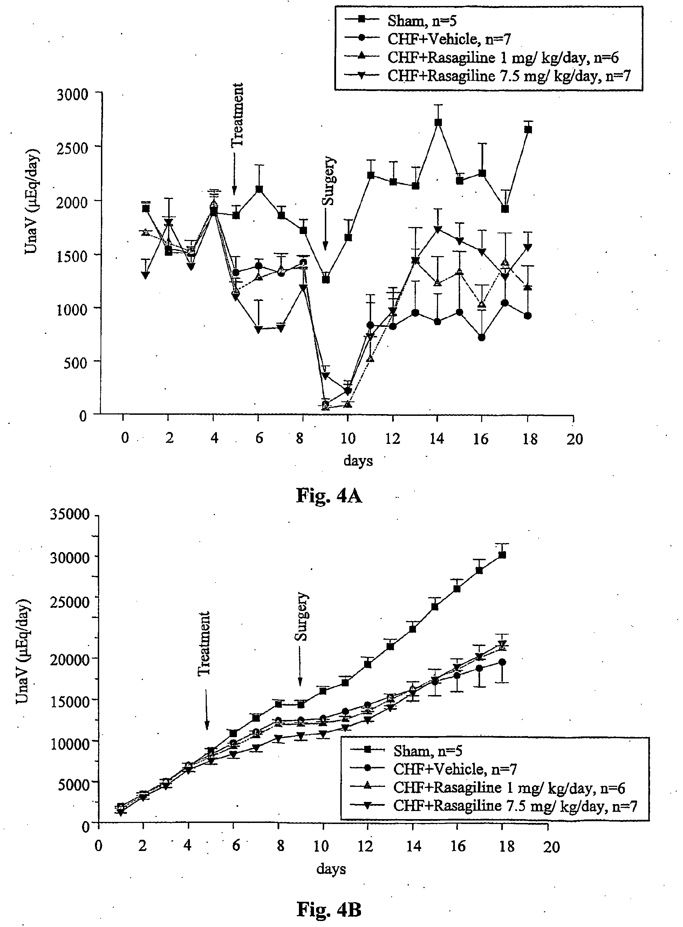

**Figs. 4A-4D** show the effects of chronic treatment with 1 or 7.5 mg/kg/day of either rasagiline or S-(-)-N-propargyl-1-aminoindan (TVP-1022) on daily (**4A, 4C**) and cumulative (**4B, 4D**) sodium (Na⁺) excretion (UNaV) in rats with CHF vs. sham operated animals. Animals were treated as described in Fig. 3 hereinabove. Rats treated with vehicle (water) served as controls (P<0.05).

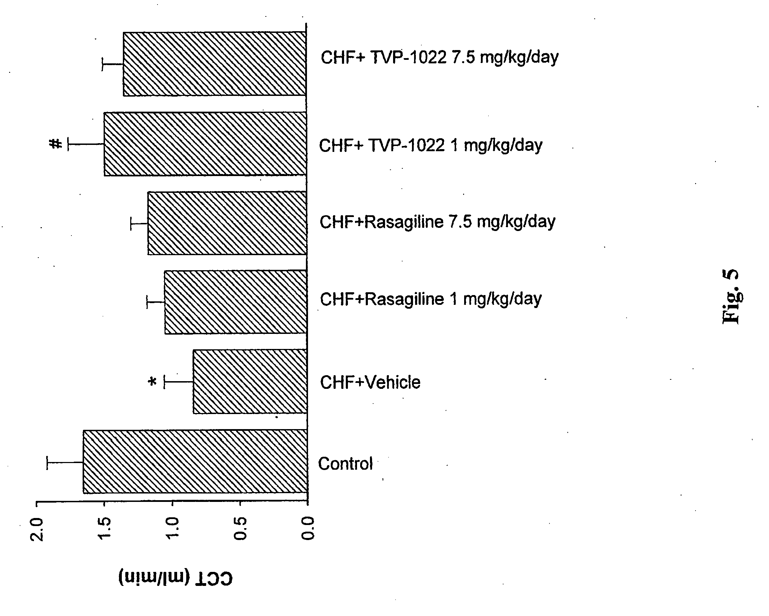

**Fig. 5** shows the effects of chronic treatment with 1 or 7.5 mg/kg/day of either rasagiline or S-(-)-N-propargyl-1-aminoindan (TVP-1022) on creatinine clearance test (CCT), in rats with CHF. Animals were treated as described in Fig. 3 hereinabove. Rats treated with vehicle (water) served as controls (* - P<0.05 vs. control; # - P<0.05 vs. untreated CHF).

The present invention relates to the use of an active agent for the preparation of a pharmaceutical composition for treatment or prevention of acute renal failure wherein said active agent is selected from S-(-)-N-propargyl-1-aminoindan, or a pharmaceutically acceptable salt thereof, and a pharmaceutically acceptable carrier.

The composition of the invention may be used for the treatment or prevention of acute renal failure (ARF), independently of its cause and of its type.

Depending on the localization or the nature of the renal insult, ARF and CRF may be classified as prerenal, renal or postrenal ARF or CRF.

ARF is a syndrome characterized by a sudden decrease of the glomerular filtration rate (GFR) and consequently an increase in blood nitrogen products (blood urea nitrogen and creatinine). Prerenal ARF may be caused, for example, by myocarditis, myocardial infarction, congestive heart failure, arrhythmia, pericardial tamponade, pulmonary embolism, decreased extracellular fluid volume, excessive diuresis, hemorrhage, vomiting, diarrhea, sepsis, peritonitis, pancreatitis, burns, etc. The condition is reversible if the underlying disease is resolved. Parenchymatous or renal ARF are caused, for example, by acute tubular necrosis or injury resulting from cardiovascular surgery, sepsis, exposure to toxic substances and nephrotoxic drugs, accumulation of endogenous nephrotoxins, hemoglobinuria, or intrarenal precipitation (hypercalcemia, urates, sulfonamides, acyclovir, myeloma protein), or by acute glomerulonephritis. Obstructive or postrenal ARF may be caused by congenital abnomalies, acquired uropathies, malignant diseases, some drugs, infections, and others.

CRF is the progressive loss of kidney function. The cause(s) of CRF may be difficult if not impossible to be determined. Prerenal conditions such as poor cardiac function, chronic liver failure, and atherosclerosis ("hardening") of the renal artery may induce ischemic nephropathy. Interference with the normal flow of urine can produce backpressure within the kidneys, damage nephrons, and lead to obstructive uropathy, a disease of the urinary tract. Abnormalities that may hamper urine flow and clause post-renal CRF include bladder outlet obstruction, kidney stones, and obstruction of the tubules. Renal CRF is caused by changes within the kidneys and include diabetic nephropathy, chronic glomerular nephritis, chronic interstitial nephritis, vasculitis, renal vascular CRF.

The active agent of the invention is used for the preparation of a pharmaceutical composition for treatment or prevention of prerenal ARF caused by congestive heart failure.

Our findings clearly demonstrate that pretreatment with S-(-)-N-propargyl-1-aminoindan, prior to the induction of renal ischemia in rats, improved the GFR of the ischemic kidney and increased the fractional excretion of Na⁺ (FENa), as shown in Example 1 hereinafter. In addition, chronic administration of S-(-)-N-propargyl-1-aminoindan to CHF rats improved the GFR by restoring it almost to normal levels, and enhanced both urinary flow rate and Na⁺ excretion as shown in Example 2.

The active agent used in the present invention is S-(-)-N-propargyl-1-aminoindan (described, for example, in US 6,277,886).

In a further preferred embodiment, the active agent is a pharmaceutically acceptable salt of S-(-)-N-propargyl-1-aminoindan including the mesylate, maleate, fumarate, tartrate, hydrochloride, hydrobromide, esylate, p-toluenesulfonate, benzoate, acetate, phosphate and sulfate salts.

Herein disclosed are further analogs of N-propargyl-1-aminoindan, an enantiomer or a pharmaceutically acceptable salt thereof. The analogs are the compounds described in US 5,486,541 such as 4-fluoro-N-propargyl-1-aminoindan, 5-fluoro-N-propargyl-1-aminoindan, 6-fluoro-N-propargyl-1-aminoindan, an enantiomer thereof and pharmaceutically acceptable addition salts thereof and the compounds described in US 6,251,938 such as (rac)-3-(N-methyl,N-propyl-carbamyloxy)-α-methyl-N'-propargyl phenethylamine HCl; (rac)-3-(N,N-dimethyl-carbamyloxy)- α-methyl-N'-methyl, N'-propargyl phenethylamine HCl; (rac)-3-(N-methyl,N-hexyl-carbamyloxy)-α-methyl-N'-methyl, N'-propargyl phenethylamine mesylate; (rac)-3-(N-methyl,N-cyclohexyl-carbamyloxy)-α-methyl-N'-methyl,N-propargylphenethyl HCl; and (S)-3-(N-methyl, N-hexyl-carbamyloxy)-α-methyl-N'-methyl,N'-propargyl phenethylamine ethane-sulfonate. Further analogs are the compounds described in US 6,303,650 such as (rac) 6-(N-methyl, N-ethyl-carbamyloxy)-N-propargyl-1-aminoindan HCl; (rac) 6-(N,N-dimethyl, carbamyloxy)-N'-methyl-N-propargyl-1-aminoindan HCl; (rac) 6-(N-methyl, N-ethyl-carbamyloxy-N'-proparoyl-1-aminotetralin HCl; (rac) 6-(N,N-dimethyl-thiocarbamyloxy)-1-aminoindan HCl; (rac) 6-(N-propyl-carbamyloxy-N'-propargyl-1-aminoindan HCl; (rac) 5-chloro-6-(N-methyl, N-propyl-carbamyloxy)-N'-propargyl-1-aminoindan HCl; (S)-6-(N-methyl), N-propyl-carbamyloxy)-N'-propargyl-1-aminoindan HCl; and (R)-6-(N-methyl, N-ethyl-carbamyloxy)-N-propargyl-1-aminoindan hemi-(L)-tartrate, and 6-(N-methyl, N-ethyl-carbamyloxy)-N'-methyl,N'-propargyl-1-aminoindan described in US 6,462,222.

Herein disclosed are further aliphatic propargylamine described in US 5,169,868, US 5,840,979 and US 6,251,950 such as the compounds N-(1-heptyl)propargylamine; N-(1-octyl)propargylamine; N-(1-nonyl) propargylamine; N-(1-decyl)propargylamine; N-(1-undecyl)propargylamine: N-(1-dodecyl) propargylamine; R-N-(2-butyl) propargylamine; R-N-(2-pentyl) propargylamine; R-N-(2-hexyl)propargylamine; R-N-(2-heptyl)propargylamine; R-N-(2-octyl) propargylamine; R-N-(2-nonyl) propargylamine; R-N-(2-decyl) propargylamine, R-N-(2-undecyl)proparaylamine; R-N-(2-dodecyl)propargylamine: N-(1-butyl)-N-methylpropargylamine; N-(2-butyl)-N-methylpropargylamine ; N-(2-pentyl)-N-methylpropargylamine; N-(1-pentyl)-N-methylpropargylamine; N-(2-hexyl)-N-methylpropargylamine; N-(2-heptyl)-N-methylpropargylamine; N-(2-decyl)-N-methylpropargylamine; N-(2-dodecyl)-N-methylpropargylamine; R(-)-N-(2-butyl)-N-methylpropargylamine; or a pharmaceutically acceptable salt thereof.

Described are further selegiline, desmethylselegiline or norprenyl, pargyline, chlorgyline and

the compound (N-methyl-N-propargyl-10-aminomethyl-dibenzo[b,f]oxepin (known as CGP 3466, described in Zimmermann *et al.,* 1999).

The pharmaceutical composition provided by the present invention may be in solid, semisolid or liquid form and may further include pharmaceutically acceptable fillers, carriers or diluents, and other inert ingredients and excipients. The composition can be administered by any suitable route, e.g. intravenously, orally, parenterally, rectally, or transdermally. The dosage will depend on the state of the patient and severity of the disease and will be determined as deemed appropriate by the practitioner.

In one embodiment, the pharmaceutically acceptable carrier is a solid and the pharmaceutical composition is in a suitable form for oral administration including tablets, compressed or coated pills, dragees, sachets, hard or soft gelatin capsules, and sublingual tablets. In a more preferred embodiment, the pharmaceutical composition is a tablet containing an amount of the active agent in the range of 0.1 - 100 mg, preferably from 1 mg to about 10 mg.

In another embodiment, the pharmaceutically acceptable carrier is a liquid and the pharmaceutical composition is an injectable solution. The amount of the active agent in the injectable solution is in the range of from about 0.1 mg/kg to 100 mg/kg, more preferably 1 mg/kg to 10 mg/kg.

For parenteral administration the invention provides ampoules or vials that include an aqueous or non-aqueous solution or emulsion. For rectal administration there are provided suppositories with hydrophilic or hydrophobic (gel) vehicles.

The dosage and frequency of administration of the drug will depend from the age and condition of the patient, type of disorder and its severity, and will be determined according to the physician's judgment. It can be presumed that for preventive treatment of subjects susceptible to renal failure lower doses will be needed while higher doses will be administered in acute cases.

In one embodiment of the invention, the active agent is administered alone. In other embodiments of the invention, the active agent is administered in combination with another known drug for treatment of renal failure, either before, simultaneously or after said other drug.

The following examples illustrate certain features of the present invention.

### EXAMPLES

### Material and Methods

***(i)*** ***Materials**.* Rasagiline and its enantiomer S(-)-N-propargyl-1-aminoindan were kindly donated by Teva Pharmaceutical Industries Ltd. (Petach Tikva, Israel).
***(ii)*** ***Animals**.* Studies were conducted on Sprague Dawley rats (Harlan Laboratories Ltd., Jerusalem, Israel), weighing 290∼330 g. The animals were kept in a temperature-controlled room, and were fed standard rat chow containing 0.5% NaCl and tap water *ad libitum.* All experiments were performed according to the guidelines of the Technion Committee for Supervision of Animal Experiments (Haifa, Israel). Rats were subjected to one of the experimental protocols, described in Examples 1 and 2 hereinafter.
*(iii) Chemical analysis.* Sodium concentration in plasma and urine was determined by flame photometry (model IL 943, Instrumentation Laboratory). Inulin and creatinine concentrations were determined by the colorimetric anthrone method. Glomerular filtration rate (GFR) was equated with the clearance of inulin or creatinine.
*(iv) Statistical analysis.* Statistical significance was assessed by one-way analysis of variance (ANOVA), ANOVA for repeated measures, or two-way ANOVA, as appropriate. The Dunnett test was used for data point comparisons of each group. P<0.05 was considered statistically significant. Data are presented as means ± S.E.M.

In the following experiments, our aim was to examine the efficacy of rasagiline (reference example) and its S-isomer, S-(-)-N-propargyl-1-aminoindan, in two different experimental protocols: (i) Renoprotective efficacy in renal ischemia; and (ii) Renal function in rats with experimental heart failure.

### Example 1. Acute studies - Effects of rasagiline or S-(-)-N-propargyl-1-aminoindan on renal clearance parameters in rats with renal ischemia

This protocol was designed to assess the reno-protective effects of rasagiline and S-(-)-N-propargyl-1-aminoindan in a rat renal ischemia-reperfusion injury (IRI) model of acute renal failure (ARF). For this purpose, the effects of either rasagiline or S-(-)-N-propargyl-1-aminoindan on renal clearance parameters, i.e., glomerular filtration rate (GFR), absolute and fractional sodium excretion rates (U_{Na}V and FE_{Na}, respectively), and urinary flow (V) were studied in rats that underwent renal ischemia. Rats that underwent the same procedure, but were treated with vehicle, served as controls.

The surgical procedure for the induction of renal ischemia has been previously described (Sela *et al*., 1992). Briefly, the animals were anesthetized with Inactin (100 mg/kg, i.p.), placed on a thermo-regulated (37°C) surgical table and prepared for hemodynamic and clearance studies (Brodsky *et al.,* 1998). After tracheotomy, polyethylene tubes (PE₅₀) were inserted into the carotid artery, jugular vein and urinary bladder for blood pressure monitoring, infusion of various solutions and urine collections, respectively. A solution of 2% inulin in 0.9% saline was infused throughout the experiment at a rate of 1.0% of body weight per hour. After surgery and a 60-minute equilibration period, two baseline clearance periods of 30 minutes each were obtained. Then, the left kidney was exposed and the renal artery was clamped for forty-five minutes of ischemia, followed by 60 min of reperfusion. The right kidney served as the control, for each animal. Thirty minutes prior to the induction of the renal ischemia, animals were treated with either rasagiline or S-(-)-N-propargyl-1-aminoindan administered as a bolus injection (1.0 mg/kg, I.V.), followed by a sustained infusion of the drugs at a dose of 2 mg/kg/h throughout the whole experiment. Animals treated with saline served as controls. Then, two additional clearance periods were obtained under the influence of the drug. Urine volume was determined gravimetrically. Blood samples were obtained in the midst of every 2^{nd} clearance period.

As shown in **Figs. 1A-1B****,** it seems that S-(-)-N-propargyl-1-aminoindan, and to a lesser extent rasagiline, improved the function of the ischemic kidney as well as the function of the control normal kidney. **Fig. 2A** shows that S-(-)-N-propargyl-1-aminoindan, and to a higher extent rasagiline, increased the fractional excretion of Na⁺ (FENa) by normal kidney. In contrast, S-(-)-N-propargyl-1-aminoindan enhanced FENa by ischemic kidney more than rasagiline **(****Fig. 2B****).**

### Example 2. Chronic studies - Effects of chronic administration of rasagiline or S-(-)-N-propargyl-1-aminoindan on urine volume and sodium excretion in CHF rats

This protocol was designed to evaluate the effects of long-term administration (14 days) of rasagiline or S-(-)-N-propargyl-1-aminoindan on renal clearance and excretory parameters in rats with experimental congestive heart failure (CHF) induced by the placement of an aortocaval fistula (ACF) between the abdominal aorta and the inferior vena-cava as described by Abassi *et al.* (2001).

In short, the abdominal aorta and inferior vena-cava were exposed through a mid-abdominal incision under pentobarbital anesthesia (60 mg/kg, I.P.), and an ACF was surgically created in the common wall of the two vessels (side to side, 1.2 mm O.D.). Following surgery, the rats were allowed to recover and housed in individual metabolic cages for measurements of daily urine volume and urinary sodium/potassium excretion.

Rasagiline or 8-(-)-N-propargyl-1-aminoindan were dissolved in drinking water and given orally at a dose of 1 or 7.5 mg/kg/day for 5 days to normal rats. Following this period, the animals were subjected either to sham operation (n=5) or to creation of aortocaval fistula (ACF) (n=6-7) as described above. These experimental groups were treated daily by gavage with 1 or 7.5 mg/kg of rasagiline or S-(-)-N-propargyl-1-aminoindan for additional 14 days, and their daily urine volume and sodium excretion were measured. Rats treated with vehicle (water) served as controls. After completion of the treatment period, animals from the different experimental groups were sacrificed by decapitation and their blood was collected in pre-cooled tubes. Concentrations of creatinine in plasma and urine samples were measured in the last day of experiment, and creatinine clearance test (CCT) was equated with the renal clearance (C_{cr}), which is equal to glumerular filtration rate (GFR). Rats with sham operation or animals with arterio-venous fistula treated with vehicle served as controls.

As previously described (Abassi *et al.,* 2001), in CHF rats both absolute and cumulative urinary volume were attenuated. **Figs. 3A-3D** show that chronic administration of low (1 mg/kg/day) or high (7.5 mg/kg/day) doses of rasagiline to rats with CHF did not affect significantly daily or cumulative urinary volume **(****Figs. 3A-3B****);** however, 7.5 mg/kg/day S-(-)-N-propargyl-1-aminoindan significantly increased both daily and cumulative urine volume **(****Figs. 3C-3D****)** (P<0.05). For example, the cumulative urine volume on day 18 was increased by 44%, from 176.6±17.6 (in CHF+vehicle) to 254.2±21.7 ml (in CHF+7.5 mg/kg/day) **(****Fig. 3D****).** At the lower dose (1 mg/kg/day), S-(-)-N-propargyl-1-aminoindan moderately increased both parameters, but the effect was not statistically significant.

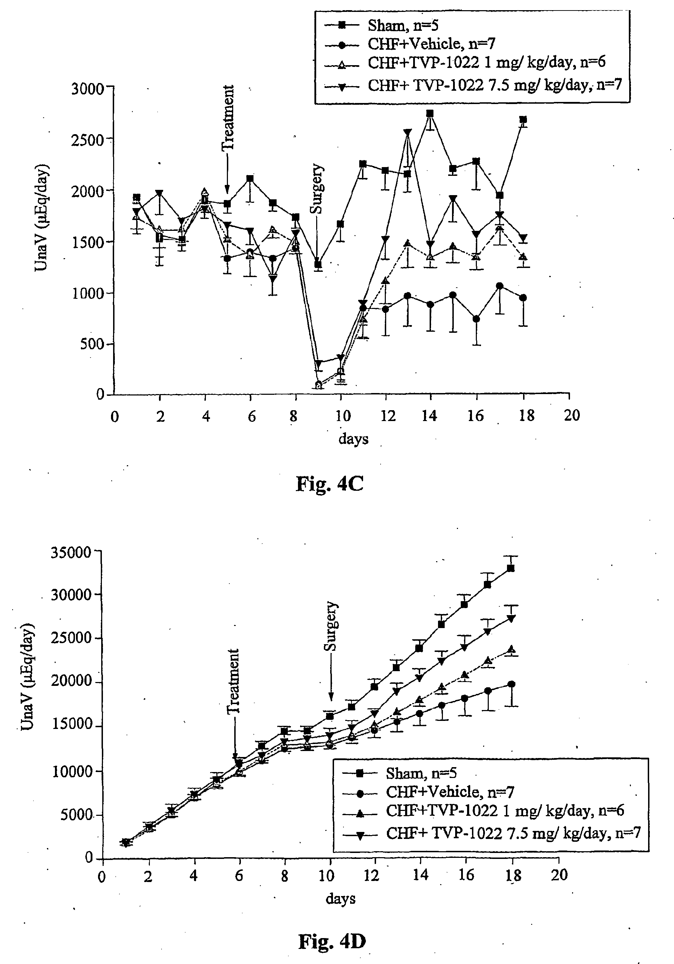

In addition to the reduction in urine volume, as shown in **Figs. 4A-4D****,** both absolute and cumulative Na⁺ excretion were reduced in CHF rats compared to the control rats (P<0.05). Although it appears that from day 12 onwards there was increase in daily Na⁺ excretion, rasagiline did not affect significantly neither daily nor cumulative Na⁺ excretion (Figs. **4A-4B****).** In contrast, both doses of S-(-)-N-propargyl-1-aminoindan increased significantly (P<0.05) daily and cumulative Na⁺ excretion **(****Figs. 4C-4D****).** For example, 7.5 mg/kg/day S-(-)-N-propargyl-1-aminoindan increased Na⁺ excretion (calculated on day 18) by 49%, from 19645.2±418.7 µEq/2 weeks (in CHF+vehicle) to 31177.1±118.2 µEq/2 weeks **(****Fig. 4D****).**

Finally, as reported by *Abassi al.* (2001), GFR was reduced in CHF rats from 1.66±0.26 ml/min (in sham controls) to 0.73±0.23 ml/min (P<0.01). In accordance with the improvement of the diuretic and natriuretic parameters by S-(-)-N-propargyl-1-aminoindan in CHF rats, S-(-)-N-propargyl-1-aminoindan at 1 and 7.5 mg/kg/day increased GFR from 0.73±0.23 ml/min to 1.49±0.26 ml/min and 1.18±0.16 ml/min, respectively, as shown in **Fig. 5****. Collectively,** these data suggest that S-(-)-N-propargyl-1-aminoindan could improve kidney function in heart failure disease state.

### REFERENCES

Abassi, Z. Haramati, A. Hoffman, A. Burnett Jr, J.C. Winaver, J., Effect of converting-enzyme inhibition on renal response to ANF in rats with experimental heart failure, Am J Physiol., 1990, 259, R84-R89
Abassi, Z.A. Brodsky, S. Karram, T. Dobkin, I. Winaver, J. Hoffman, A., Temporal changes in natriuretic and antinatriuretic systems after closure of a large arteriovenous fistula, Cardiovasc Res., 2001, 51, 567-576
Am, O.B. Amit, T. Youdim, M.B.H., Contrasting neuroprotective and neurotoxic actions of respective metabolites of anti-Parkinson drugs rasagiline and selegiline, Neurosci. Lett., 2004, 355, 169-172
Arendshorst, W.J. Finn, W.F. Gottschalk, C.W., Pathogenesis of acute renal failure following temporary renal ischemia in the rat, Circ Res, 1975, 37, 558-568
Birkmayer, W. Riederer, P. Youdim, M.B.H. Linauer, W., The potentiation of anti-akinetic effect after L-dopa treatment by an inhibitor of MAO-B deprenyl, J. Neural. Transm., 1975, 36, 303-326
Brodsky, S. Gurbanov, K. Abassi, Z. Hoffman, A. Ruffolo Jr, R.R. Feuerstein, G.Z. Winaver, J., Effects of eprosartan on renal function and cardiac hypertrophy in rats with experimental heart failure, Hypertension, 1998, 32, 746-752
Brooks, D.J. Sagar, H., UK-Irish Entacaopone Study Group. Entacapone is beneficial in both fluctuating and non-fluctuating patients with Parkinson's disease: a randomized, placebo controlled, double blind, six month study, J. Neurol. Neurosurg. Psychiatry, 2003, 74, 1071-1079
Canavese, C. Strattaand, P. Vercellone, A., The case for oxygen free radicals in the pathogenesis of ischemic acute renal failure, Nephron, 1988, 49, 9-15
Cristol, J.P. Thiemermann, C. Mitchell, A. Walder, C. Vane, J.R., Support of renal blood flow after ischemic-reperfusion injury by endogenous formation of nitric oxide and of cyclo-oxygenase vasodilator metabolites, Br J Pharmacol, 1993, 109, 188-194
Dzau, V.J., Renal and circulatory mechanisms in congestive heart failure, Kidney Int., 1987, 31,1402-1415
Finberg, J.P. Ari, G. Lavian, G. Hovevey-Sion, D., Modification of alpha-2 presynaptic receptor activity and catecholamine release following chronic MAO inhibition, J Neural Transm Suppl., 1990, 32, 405-412
Finberg, J.P.M. Lamensdorf, I. Weinstock, M. Schwartz, M. Youdim, M.B.H., Pharmacology of rasagiline (N-propargyl-IR-aminoindan), Adv. Neurol., 1999, 80, 495-501
Glezer, S. Finberg, J.P., Pharmacological comparison between the actions of methamphetamine and 1-aminoindan stereoisomers on sympathetic nervous function in rat vas deferens, Eur. J. Pharmacol., 2003, 472, 173-177
Green, J. Abassi, Z. Winaver, J. Skorecki, K., Acute renal failure: clinical and pathophysiological aspects. In The Kidney: Physiology and Pathophysiology, 3rd edition, 2000, edited by D. W. Seldin and G. Giebisch. Philadelphia: Lippincott-Raven
Greene, E.L. Paller, M.S., Oxygen free radicals in acute renal failure. Miner Electrolyte Metab., 1991, 17, 124-132
Hillege, H.L. Girbes, A.R. de Kam, P.J. Boomsma, F. de Zeeuw, D. Charlesworth, A. Hampton, J.R. van Veldhuisen, D.J., Renal function, neurohormonal activation, and survival in patients with chronic heart failure, Circulation, 2000, 102, 203-210
Hostetter, T.H. Pfeffer, J.M. Pfeffer, M.A. Dworkin, L.D. Braunwald, E. Brenner, B.M., Cardiorenal hemodynamics and sodium excretion in rats with myocardial infarction. Am J Physiol., 1983, 245, H98-H103
Kraemer, T. Maurer, H.H., Toxicokinetics of amphetamines: metabolism and toxicokinetic data of designer drugs, amphetamine, methamphetamine, and their N-alkyl derivatives. Ther. Drug. Monit., 2002, 24, 277-289
Kribben, A. Edelstein, C.L. Schrier, R.W., Pathophysiology of acute renal failure. J. Nephrol., 1999, 12 (Suppl. 2), S142-S151
< Eliash, S., Speiser, Z., Cohen, S., Rasagiline and its (S)enantiomer increase survival and prevent stroke in salt-loaded stroke-prone spontaneously hypertensive rats, J. Neural Transm., 2001, 108, 909-923 >
Lavian, G. Finberg, J.P. Youdim, M.B.H., The advent of a new generation of monoamine oxidase inhibitor antidepressants: pharmacologic studies with moclobemide and brofaromine, Clin. Neuropharmacol., 1993, 16 (Suppl. 2), S1-7
Lees, A. Show, K.M. Kahout, L.J. Stem, G. Elsworth, J.D. Sandler, M. Youdim, M.B.H., Deprenyl in Parkinson's Disease, Lancet, 1977, 2, 791-795
Lees, A.J., Comparison of therapeutic effects and mortality data of levodopa and levodopa combined with selegiline in patients with early, mild Parkinson's disease. Parkinson Disease Research Group of the United Kingdom, Br. Med. J., 1995, 311, 1601-1607
Mandel, S. Grunblatt, E. Riederer, P. Gerlach, M. Levites, Y. Youdim, M.B.H., Neuroprotective strategies in Parkinson's disease: an update on progress, CNS. Drugs., 2003, 17, 729-762
Parkinson Study Group (Datatop), Selegiline and Parkinson's disease. N. Engl. J. Med., 1989, 321, 1364-1371
Schrier, R.W. Gurevich, A.K. Cadnapaphomchai, M.A., Pathogenesis and management of sodium and water retention in cardiac failure and cirrhosis, Semin Nephrol., 2001, 21, 157-172
Schrier, R.W. Wang, W. Poole, B. Mitra, A., Acute renal failure: definitions, diagnosis, pathogenesis and therapy, J. Clin. Invest., 2004, 114, 5-14
Shin, H.S., Metabolism of selegiline in humans: Identification, excretion and stereochemistry of urine metabolites, Drug. Metab. Dispos., 1997, 25, 657-662
Simpson, L.L., Evidence that deprenyl, a type B monoamine oxidase inhibitor is an indirectly acting sympathomimetic amine, Biochem. Pharmacol., 1978, 27, 1591-1595
Youdim, M.B.H. Gross, A. Finberg, J.P., Rasagiline [N-propargyl-1R(+)-aminoindan], a selective and potent inhibitor of mitochondrial monoamine oxidase B, Br. J. Pharmacol., 2001, 132, 500-506
Youdim, M.B.H., Rasagiline: an antiparkinson drug with neutroprotective activity, Expert. Rev., Neurotherapeutics, 2003, 3, 737-749

## Claims

1. Use of an active agent for the preparation of a pharmaceutical composition for treatment or prevention of acute renal failure (ARF), wherein said active agent is selected from S-(-)-N-propargyl-1-aminoindan, or a pharmaceutically acceptable salt thereof.

2. The use of claim 1, wherein said active agent is S-(-)-N-propargyl-1-aminoindan.

3. The use of claim 1, wherein said active ingredient is a pharmaceutically acceptable salt of S-(-)-N-propargyl-1-aminoindan.

4. The use of claim 3, wherein said pharmaceutically acceptable salt is selected from the mesylate salt; the esylate salt; the sulfate salt; the maleate salt; the fumarate salt; the tartrate salt; the hydrobromide salt; the p-toluenesulfonate salt; the benzoate salt; the acetate salt; the phosphate salt; and the hydrochloride salt of S-(-)-N-propargyl-1-aminoindan.

5. The use of claim 1, wherein said ARF is caused by congestive heart failure.

6. A pharmaceutically active agent selected from S-(-)-N-propargyl-1-aminoindan, or a pharmaceutically acceptable salt thereof, for use in treating or preventing acute renal failure (ARF).

7. The pharmaceutically active agent as defined in claim 6 for the use as defined in claim 6, wherein said ARF is caused by congestive heart failure.

## Patentansprüche

1. Verwendung eines Wirkstoffs zur Herstellung einer pharmazeutischen Zusammensetzung zur Behandlung oder Prävention von akutem Nierenversagen (ANV), wobei der Wirkstoff ausgewählt ist aus S-(-)-N-Propargyl-1-aminoindan oder einem pharmazeutisch annehmbaren Salz davon.

2. Verwendung nach Anspruch 1, wobei der Wirkstoff S-(-)-N-Propargyl-1-aminoindan ist.

3. Verwendung nach Anspruch 1, wobei der Wirkstoff ein pharmazeutisch annehmbares Salz von S-(-)-N-Propargyl-1-aminoindan ist.

4. Verwendung nach Anspruch 3, wobei das pharmazeutisch annehmbare Salz ausgewählt ist aus Mesylatsalz; Esylatsalz; Sulfatsalz; Maleatsalz, Fumaratsalz; Tartratsalz; Hydrobromidsalz; p-Toluensulfonatsalz; Benzoatsalz; Acetatsalz, Phosphatsalz; und Hydrochloridsalz von S-(-)-N-Propargyl-1-aminoindan.

5. Verwendung nach Anspruch 1, wobei das ANV durch eine kongestive Herzinsuffizienz verursacht ist.

6. Pharmazeutischer Wirkstoff, ausgewählt aus S-(-)-N-Propargyl-1-aminoindan oder einem pharmazeutisch annehmbaren Salz davon, zur Verwendung bei der Behandlung oder Prävention von akutem Nierenversagen (ANV).

7. Pharmazeutischer Wirkstoff, wie in Anspruch 6 definiert, zur Verwendung, wie in Anspruch 6 definiert, wobei das ANV durch eine kongestive Herzinsuffizienz verursacht ist.

## Revendications

1. Utilisation d'un agent actif pour la préparation d'une composition pharmaceutique pour le traitement ou la prévention de l'insuffisance rénale aiguë (IRA), dans laquelle ledit agent actif est choisi parmi le S-(-)-N-propargyl-1-amino-indane ou un sel pharmaceutiquement acceptable de celui-ci.

2. Utilisation selon la revendication 1, dans laquelle ledit agent actif est le S-(-)-N-propargyl-1-amino-indane.

3. Utilisation selon la revendication 1, dans laquelle ledit ingrédient actif est un sel pharmaceutiquement acceptable du S-(-)-N-propargyl-1-amino-indane.

4. Utilisation selon la revendication 3, dans laquelle ledit sel pharmaceutiquement acceptable est choisi parmi le sel de mésylate ; le sel d'ésylate ; le sel de sulfate ; le sel de maléate ; le sel de fumarate ; le sel de tartrate ; le sel de bromhydrate ; le sel de p-toluène-sulfonate ; le sel de benzoate ; le sel d'acétate ; le sel de phosphate ; et le sel de chlorhydrate du S-(-)-N-propargyl-1-amino-indane.

5. Utilisation selon la revendication 1, dans laquelle ladite IRA est provoquée par une insuffisance cardiaque congestive.

6. Agent pharmaceutiquement actif choisi parmi le S-(-)-N-propargyl-1-amino-indane ou un sel pharmaceutiquement acceptable de celui-ci, pour une utilisation dans le traitement ou la prévention de l'insuffisance rénale aiguë (IRA).

7. Agent pharmaceutiquement actif selon la revendication 6, pour l'utilisation selon la revendication 6, ladite IRA étant provoquée par une insuffisance cardiaque congestive.
